# EUROPEAN PATENT APPLICATION

(11) **EP 4 483 710 A1**
(43) Date of publication of application: **01.01.2025**
(21) Application number: 23760030.9
(22) Date of filing: 22.02.2023
(51) Int. Cl.: A01H 1/04, C12N 15/29, C12Q 1/68, A01H 6/82

(54) **LOW-REVERSION TYPE TOBACCO PLANT HAVING LOW NORNICOTINE CONTENT, METHOD FOR SELECTING SAME, AND METHOD FOR PRODUCING TOBACCO PLANT LINE**

(30) Priority: 24.02.2022 JP 2022027284
(71) Applicant: Japan Tobacco Inc., Tokyo 105-6927 (JP)
(72) Inventor: TAKAKURA, Yoshimitsu, Tokyo 130-8603 (JP); TAJIMA, Tomoyuki, Tokyo 130-8603 (JP); YOSHIKIYO, Tsubasa, Tokyo 130-8603 (JP); TAKEUCHI, Takanori, Tokyo 130-8603 (JP)
(74) Representative: Hoffmann Eitle
(86) International application number: PCT/JP2023/006427
(87) International publication number: WO 2023/163031

(57) **Abstract**

Provided is a method for producing a low reversion type low nornicotine content tobacco plant line without use of a genetic modification technique or a factitious mutant preparation technique. Also provided is a tobacco plant. In the method in accordance with the present invention, a variety-specific DNA sequence that links with a low reversion type low nornicotine character is determined, and selection is carried out with use of the sequence as an index.

## Description

### Technical Field

The present invention relates to a low reversion type low nornicotine content tobacco plant, a method for selecting the low reversion type low nornicotine content tobacco plant, and a method for producing a tobacco plant line.

### Background Art

Nornicotine is a precursor of N-nitrosonornicotine (NNN), which is a type of tobacco-specific nitrosamine (TSNA). A character in which nornicotine is stably kept at a low level is important for tobacco breeding. NNN is produced in such a manner that nicotine is converted into nornicotine by nicotine N-demethylase (NND) and then the nornicotine nonenzymatically reacts with nitrous acid or the like. NND genes form a gene family, and three types of NND (CYP82E4, CYP82E5, and CYP82E10) function in *Nicotiana tabacum.* Among these three types of NND, a main gene is one that encodes CYP82E4, which is induced in a senescent (mature) leaf and a leaf in the process of curing (Non-Patent Literature 1), and conversion by CYP82E4 accounts for 98% of production of nornicotine. Out of an S-type genome derived from *Nicotiana sylvestris* and a T-type genome derived from *Nicotiana tomentosiformis,* CYP82E4 in *Nicotiana tabacum,* which is an amphidiploid, is encoded by the T-type genome. There have been known tobacco breeding methods which focus on expression of the NND genes. For example, it is known that tobacco having a reduced NNN content can be produced by low nornicotine breeding in which a mutation of a CYP82E4 gene is used as an index (Patent Literature 1), RNAi of an NND gene (Non-Patent Literature 2), or selection of an ethylmethane sulfonate (EMS) tobacco knockout mutant of an NND gene (Non-Patent Literatures 3 and 4). It is also known that the promoter region of the CYP82E4 gene, among the NND genes, has 12 repeats of guanine-adenine (Patent Literatures 1 and 2).

### Citation List

### [Patent Literature]

[Patent Literature 1]
   Japanese Patent Application Publication Tokukai No. 2008-506362
[Patent Literature 2]
   Japanese Patent Application Publication Tokukai No. 2013-027393

### [Non-patent Literature]

[Non-patent Literature 1]
   Siminszky et al. (2005) Conversion of nicotine to nornicotine in Nicotiana tabacum is mediated by CYP82E4, a cytochrome P450 monooxygenase. Proc. Natl. Acad. Sci. U. S. A. 102: 14919-14924.
[Non-patent Literature 2]
   Lewis et al. (2008) RNA interference (RNAi)-induced suppression of nicotine demethylase activity reduces levels of a key carcinogen in cured tobacco leaves. Plant Biotechnol. J. 6: 346-354.
[Non-patent Literature 3]
   Lewis et al. (2010) Three nicotine demethylase genes mediate nornicotine biosynthesis in Nicotiana tabacum L.: functional characterization of the CYP82E10 gene. Phytochemistry 71: 1988-1998.
[Non-patent Literature 4]
   Julio et.al. (2008) Reducing the content of nornicotine in tobacco via targeted mutation breeding. Mol. Breed. 21: 369-381.

### Summary of Invention

### Technical Problem

As raw materials of tobacco products, tobacco plant individuals in each of which a nornicotine content is kept at a low level are often selected (so-called low converter type varieties). However, as generations pass, an individual having a high nornicotine content (hereinafter also referred to as "high nornicotine type converter) problematically appears at a certain frequency. However, a cause of appearance of such an individual is not well known. Meanwhile, in each of the foregoing techniques for producing tobacco having a reduced NNN content, a structural gene itself is manipulated. Therefore, it is considered that, in an RNAi mutant and a knockout mutant of the CYP82E4 gene, a high nornicotine type converter is unlikely to appear even in a case where generations pass. However, in order to obtain these tobacco plants, it is essential to use a genetic modification technique or a factitious mutant preparation technique. Moreover, in a case where the mutant preparation technique is used, a produced individual is likely to have an undesired background mutation.

An object of an aspect of the present invention is to make it possible to produce a low reversion type low nornicotine content tobacco variety in which a high nornicotine type converter is unlikely to appear even in a case were generations pass, on the basis of a conventional breeding method without use of a genetic modification technique or a factitious mutant preparation technique.

### Solution to Problem

In order to attain the above object, the inventors of the present invention conducted diligent studies, and consequently found that Matsukawa (Kanto), which is a Japanese local tobacco variety, is a tobacco variety in which a high nornicotine type converter is unlikely to appear even in a case where generations pass. Next, the inventors of the present invention found that this low reversion type low nornicotine character links with genomic DNA sequences specific to Matsukawa (Kanto) and that, by using these DNA sequences as indexes, it is possible to select a low reversion type low nornicotine content tobacco plant.

That is, in an aspect of the present invention, from a bred progeny line obtained by crossing the variety Matsukawa (Kanto) of *Nicotiana tabacum* and another variety of *Nicotiana tabacum* or a mutant of the another variety, a low reversion type low nornicotine content tobacco plant which has a low reversion type low nornicotine character that links with a genomic DNA sequence specific to Matsukawa (Kanto) is selected.

Moreover, in an aspect of the present invention, a low reversion type low nornicotine content tobacco plant which has an endogenous CYP82E4 gene that encodes an amino acid represented by SEQ ID NO. 2 is selected.

### Advantageous Effects of Invention

In an aspect of the present invention, it is possible to produce a low reversion type low nornicotine content tobacco variety, without use of a genetic modification technique and without causing an undesired background mutation which is a problem with a case where a mutant is used.

### Brief Description of Drawings

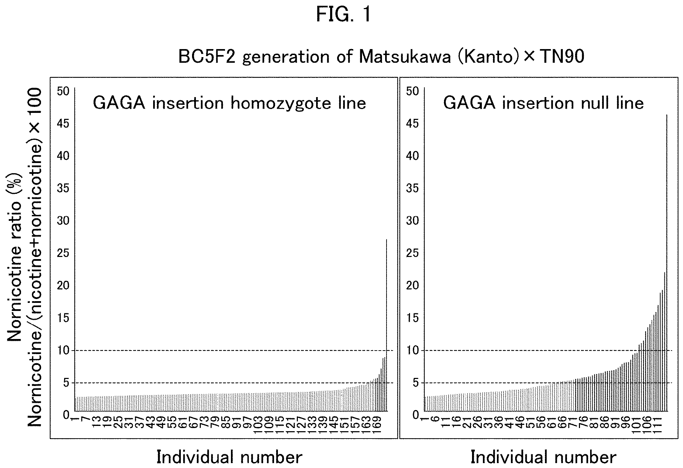

Fig. 1 illustrates graphs showing results of analyzing nornicotine ratios of a cross progeny (BC5F2) line of Matsukawa (Kanto) and TN90 in Examples.

### Description of Embodiments

The following description will discuss embodiments of the present invention in detail. The present invention is not limited to the embodiments, but can be altered by a skilled person in the art within the scope of the claims. The present invention also encompasses, in its technical scope, any embodiment derived by combining technical means disclosed in differing embodiments.

### [Method for selecting low reversion type low nornicotine content tobacco plant]

An embodiment of the present invention provides a method for selecting a low reversion type low nornicotine content tobacco plant from a bred progeny line obtained by crossing Matsukawa (Kanto), which is a Japanese local variety of *Nicotiana tabacum,* and another variety of *Nicotiana tabacum* or a mutant of the another variety.

As used herein, the expression "low reversion type" means a character in which, in a case where crossing is performed within a variety and even in a case where generations pass, an individual having a large amount of nornicotine is unlikely to appear. Specifically, the expression "low reversion type" means the following character: the number of individuals in each of which a nornicotine ratio, that is, the ratio of a nornicotine content to the total of the nornicotine content and a nicotine content is more than 5% is less than 10% and preferably less than 5% in a variety or a line; or the number of individuals in each of which the nornicotine ratio is more than 10% is less than 5% and preferably less than 2% in the variety or the line.

The "Japanese local variety" is a generic name for tobacco varieties which have been grown in each place in Japan since before flue-cured varieties, Burley varieties, and the like were introduced and improved tobacco varieties which are based on these tobacco varieties. Matsukawa (Kanto) is one of Japanese local varieties, and is an ameliorant variety for paper-wrapped cigarettes. Note that the variety Matsukawa includes a number of types of varieties such as Matsukawa (Kanto) and Matsukawa (Fukushima). Generally, in a case where merely the term "Matsukawa" is used, the term "Matsukawa" often refers to "Matsukawa (Fukushima)", not "Matsukawa (Kanto)". As shown in Examples, "Matsukawa (Kanto)" and "Matsukawa (Fukushima)" can be distinguished with use of a marker described later. As Matsukawa, the following three types are registered in the accession list of the United States Department of Agriculture (USDA): Kanto (Matsukawa): TI 1587; Kanto 201 (Matsukawa): TI 1588; and Matsukawa: TI 168. Among these three types, "Kanto (Matsukawa): TI 1587" is "Matsukawa (Kanto)".

The another variety of *Nicotiana tabacum* which is crossed with Matsukawa (Kanto) only needs to be *Nicotiana tabacum* other than Matsukawa (Kanto), and may be, but is not limited to, a cultivar of *Nicotiana tabacum* which includes the Burley varieties, the flue-cured varieties, and the Japanese local varieties. The another variety may be a variety having a relatively low conversion rate of nicotine to nornicotine (hereinafter also referred to as "low converter type") or may be a variety having a relatively high conversion rate of nicotine to nornicotine (hereinafter also referred to as "high converter type"). Even in a low converter type variety, appearance of a high nornicotine type converter is seen substantially at a certain frequency. Therefore, for commercial use, seed selection is carried out in accordance with a nornicotine content. In a high converter type variety, almost all individuals become high nornicotine type converters. The another variety may be a mutant having, in its genome, a desired mutation which confers a specific character on an individual.

A method for selecting a low reversion type low nornicotine content tobacco plant includes a step of selecting an individual having, as a homozygote, at least one marker which is in a genome sequence and which is present at a position that links with a low reversion type low nornicotine character. The at least one marker is a marker based on single nucleotide variation or insertion or deletion each of which corresponds to a specific position in a genome sequence of *Nicotiana tabacum.* Markers used in the method in accordance with the present embodiment are shown in Tables 1 and 2 below. The following description will discuss the markers.

**[Table 1]**

| Marker ID | Positions on Nt09 | Base | Marker ID | Positions on Nt09 | Base | Marker ID | Positions on Nt09 | Base |
|---|---|---|---|---|---|---|---|---|
| SNV1 | 101,496,032 | C | SNV33 | 101,725,448 | A | SNV65 | 101,393,439 | T |
| SNV2 | 101,505,318 | T | SNV34 | 101,725,867 | C | SNV66 | 101,902,591 | C |
| SNV3 | 101,508,443 | C | SNV35 | 101,727,812 | C | SNV67 | 101,903,020 | T |
| SNV4 | 101,523,017 | G | SNV36 | 101,737,537 | G | SNV63 | 101,906,181 | G |
| SNV5 | 101,524,311 | T | SNV37 | 101,737,694 | A | SNV69 | 101,908,076 | T |
| SNV6 | 101,526,270 | G | SNV33 | 101,740,101 | C | SNV70 | 101,910,271 | T |
| SNV7 | 101,526,729 | A | SNV39 | 101,742,957 | T | SNV71 | 101,914,414 | A |
| SNV8 | 101,544,000 | G | SNV40 | 101,744,375 | G | SNV72 | 101,916,403 | A |
| SNV9 | 101,545,638 | G | SNV41 | 101,748,913 | T | SNV73 | 101,917,044 | A |
| SNV10 | 101,550,197 | A | SNV42 | 101,751,457 | T | SNV74 | 101,918,202 | G |
| SNV11 | 101,562,465 | G | SNV43 | 101,752,576 | G | SNV75 | 101,918,533 | T |
| SNV12 | 101,569,477 | T | SNV44 | 101,754,559 | T | SNV76 | 101,925,101 | G |
| SNV13 | 101,572,171 | C | SNV45 | 101,777,902 | A | SNV77 | 101,933,909 | T |
| SNV14 | 101,578,805 | C | SNV 46 | 101,786,139 | G | SNV78 | 101,952,245 | G |
| SNV15 | 101,579,509 | A | SNV47 | 101,786,341 | T | SNV79 | 101,968,528 | A |
| SNV16 | 101,582,099 | G | SNV 43 | 101,787,252 | C | SNV80 | 101,976,846 | C |
| SNV17 | 101,583,478 | T | SNV49 | 101,803,382 | A | SNV81 | 101,988,964 | T |
| SNV13 | 101,584,177 | T | SNV50 | 101,822,302 | T | SNV82 | 101,989,567 | A |
| SNV19 | 101,586,295 | T | SNV51 | 101,825,828 | A | SNV83 | 101,989,748 | G |
| SNV20 | 101,586,769 | C | SNV52 | 101,826,725 | T | SNV84 | 101,992,887 | T |
| SNV21 | 101,586,820 | A | SNV53 | 101,838,779 | G | SNV85 | 101,992,988 | C |
| SNV22 | 101,614,452 | A | SNV54 | 101,843,000 | C | SNV86 | 102,013,873 | T |
| SNV23 | 101,636,959 | T | SNV55 | 101,359,933 | C | SNV87 | 102,016,365 | T |
| SNV24 | 101,637,377 | A | SNV56 | 101,863,951 | A | SNV88 | 102,018,158 | T |
| SNV25 | 101,640,243 | C | SNV57 | 101,867,595 | C | SNV89 | 102,037,650 | A |
| SNV26 | 101,643,526 | C | SNV58 | 101,870,376 | C | SNV90 | 102,039,954 | G |
| SNV27 | 101,651,143 | G | SNV59 | 101,874,929 | C | SNV91 | 102,041,266 | A |
| SNV28 | 101,669,996 | T | SNV60 | 101,375,101 | A | SNV92 | 102,053,413 | C |
| SNV29 | 101,697,595 | T | SNV61 | 101,878,001 | T | SNV93 | 102,061,071 | G |
| SNV30 | 101,698,257 | G | SNV62 | 101,886,773 | G | SNV94 | 102,067,002 | A |
| SNV31 | 101,718,066 | T | SNV63 | 101,892,276 | A | SNV95 | 102,089,629 | A |
| SNV32 | 101,721,614 | C | SNV64 | 101,893.204 | C | | | |

**[Table 2]**

| Marker ID | Positions on Nt09 | Insertion/ deletion | Marker ID | Positions on Nt09 | Insertion! deletion |
|---|---|---|---|---|---|
| InDel1 | 101544774^101544775 | A insertion | InDel12 | 101,897,361 | Deletion |
| InDel2 | 101561948^101561949 | AT insertion | InDel13 | 101,916,401 | No insertion /deletion |
| InDel3 | 101564173^101564174 | TT insertion | InDel14 | 101925961^101925962 | TT insertion |
| InDel4 | 101685327^101685328 | GAGA insertion | InDel15 | 101953087^101953088 | No insertion /deletion |
| InDel5 | 101,822,299 | Deletion | InDel16 | 101953185^101953186 | T insertion |
| InDel6 | 101,855,430 | Deletion | InDel17 | 101974552^101974553 | A insertion |
| InDel7 | 101867656^101867657 | AA insertion | InDel18 | 102003223^102003224 | A insertion |
| InDel8 | 101870399^101870400 | TTTTTTT insertion | InDel19 | 102040788..102040807 | Deletion |
| InDel9 | 101873449^101873450 | T insertion | InDel20 | 102,048,990 | No insertion /deletion |
| InDel10 | 101,881,218 | Deletion | InDel21 | 102073969^102073970 | No insertion /deletion |
| InDel11 | 101,884,256 | Deletion | | | |

Table 1 shows markers each based on single nucleotide variation on a genome (hereinafter also referred to as "SNV marker"). Table 2 shows markers each based on insertion or deletion on the genome (hereinafter also referred to as "InDel marker"). The positions of each marker on the genome, which are shown in Tables 1 and 2, are shown as positions on a chromosome Nt09 in a reference genome sequence of the variety K326 of *Nicotiana tabacum.* Note, here, that the reference genome sequence of K326 referred to herein is a sequence which was constructed by Edwards et al., 2017. BMC Genomics, 18(1), 1-14 (DOI:10.1186/s12864-017-3791-6), which is published in Sol Genomics Network (https://solgenomics.net/), and which is published in URL:ftp://ftp.solgenomics.net/genomes/Nicotiana_tabacum/edwards_et_al_20 17/assembly/Nitab-v4.5_genome_Chr_Edwards2017.fasta (last updated: April 13, 2017).

The "Base" shown in Table 1 shows the types of bases present at positions which are in a genome sequence of Matsukawa (Kanto) and which correspond to "Positions on Nt09". The markers shown in Table 1 are each a marker with use of which Matsukawa (Kanto) and at least one of K326, which is one of representative flue-cured varieties, and TN90, which is one of representative Burley varieties can be distinguished.

Table 3 below shows bases at each SNV marker site in K326, TN90, and Matsukawa (Kanto). In Table 3, a base of TN90 at each marker site is determined with reference to published sequence data (accession no. SRR954964 in the Sequence Read Archive (SRA) of GenBank). A base of K326 at each marker site is not based on the foregoing K326 reference genome sequence, but is determined with reference to another published sequence data (accession no. SRR 955769 in the Sequence Read Archive (SRA) of GenBank). Note that, in the present specification, the type of a base, the presence or absence of deletion or insertion, or the state of the deletion or the insertion at each marker site in each variety is also referred to as "genotype" of the variety.

For example, a base corresponding to SNV1 is "C" in Matsukawa (Kanto), and is "T" in each of K326 and TN90. Therefore, this marker can be used to distinguish the genotype of Matsukawa (Kanto) from the genotypes of K326 and TN90. A base corresponding to SNV3 is "C" in Matsukawa (Kanto), is "C" in K326, and is "T" in TN90. Therefore, this marker can be used to distinguish the genotype of Matsukawa (Kanto) from the genotype of TN90. A base corresponding to SNV6 is "G" in Matsukawa (Kanto), is "A" in K326, and is "G" in TN90. Therefore, this marker can be used to distinguish the genotype of Matsukawa (Kanto) from the genotype of K326.

**[Table 3]**

| Marker ID | Base | | | Marker ID | Base | | |
|---|---|---|---|---|---|---|---|
| | K326 | TN90 | Matsukawa (Kanto) | | K326 | TN90 | Matsukawa (Kanto) |
| SNV1 | T | T | C | SNV49 | C | C | A |
| SNV2 | C | C | T | SNV50 | G | G | T |
| SNV3 | C | T | C | SNV51 | G | G | A |
| SNV4 | C | C | G | SNV52 | C | C | T |
| SNV5 | C | C | T | SNV53 | A | A | G |
| SNV6 | A | G | G | SNV54 | T | C | C |
| SNV7 | G | G | A | SNV55 | T | C | C |
| SNV8 | A | A | G | SNV56 | G | G | A |
| SNV9 | A | A | G | SNV57 | T | C | C |
| SNV10 | C | C | A | SNV53 | T | C | C |
| SNV11 | A | A | G | SNV59 | G | G | C |
| SNV12 | G | G | T | SNV60 | G | G | A |
| SNV13 | C | A | C | SNV61 | C | C | T |
| SNV14 | C | A | C | SNV62 | G | A | G |
| SNV15 | T | T | A | SNV63 | C | A | A |
| SNV16 | A | A | G | SNV64 | T | C | C |
| SNV17 | C | C | T | SNV65 | C | T | T |
| SNV18 | C | C | T | SNV66 | T | C | C |
| SNV19 | A | A | T | SNV67 | G | G | T |
| SNV20 | T | T | C | SNV63 | T | G | G |
| SNV21 | A | G | A | SNV69 | A | A | T |
| SNV22 | G | G | A | SNV70 | C | C | T |
| SNV23 | C | C | T | SNV71 | G | A | A |
| SNV24 | G | G | A | SNV72 | A | T | A |
| SNV25 | A | A | C | SNV73 | A | T | A |
| SNV26 | C | T | C | SNV74 | A | A | G |
| SNV27 | A | A | G | SNV75 | C | C | T |
| SNV28 | C | C | T | SNV76 | A | A | G |
| SNV29 | C | T | T | SNV77 | C | C | T |
| SNV30 | T | T | G | SNV73 | T | T | G |
| SNV31 | C | C | T | SNV79 | G | G | A |
| SNV32 | G | G | C | SNV80 | T | T | C |
| SNV33 | G | G | A | SNV81 | C | T | T |
| SNV34 | T | T | C | SNV82 | G | G | A |
| SNV35 | A | A | C | SNV83 | A | G | G |
| SNV36 | T | T | G | SNV84 | A | A | Σ |
| SNV37 | G | G | A | SNV85 | T | T | C |
| SNV38 | C | A | C | SNV86 | G | G | T |
| SNV39 | C | C | T | SNV87 | C | C | T |
| SNV40 | T | T | G | SNV88 | C | C | T |
| SNV41 | C | C | T | SNV89 | C | A | A |
| SNV42 | T | C | T | SNV90 | G | T | G |
| SNV43 | A | G | G | SNV91 | A | T | A |
| SNV44 | G | G | T | SNV92 | T | T | C |
| SNV45 | G | G | A | SNV93 | A | A | G |
| SNV46 | C | G | G | SNV94 | G | G | A |
| SNV47 | C | C | T | SNV95 | T | T | A |
| SNV43 | T | C | C | | | | |

The "Insertion/deletion" shown in Table 2 shows the presence or absence of insertion or deletion at positions which are in the genome sequence of Matsukawa (Kanto) and which correspond to "Positions on Nt09", in comparison to the foregoing reference genome. The markers shown in Table 2 are each a marker with use of which Matsukawa (Kanto) and at least one of the flue-cured variety K326 and the Burley variety TN90 can be distinguished. Note that, in "Positions on Nt09" in Table 2, the symbol "^", for example, "101544774^101544775" represents a state (the presence or absence of insertion) between the 101544774th base and the 101544775th base of Nt09 in the reference genome. Note also that the symbol "..", for example, "102040788..102040807" represents (the presence or absence of deletion of) the 102040788th base to the 102040807 base of Nt09 in the reference genome.

Table 4 below shows states of each InDel marker site in K326, TN90, and Matsukawa (Kanto). Note that, also in Table 4, the same data as in Table 3 is referred to for genome sequence information of K326 and TN90. For example, at a position corresponding to InDel1, insertion of a base "A" is seen in Matsukawa (Kanto), and no insertion or deletion is seen in K326 and TN90. Therefore, this marker can be used to distinguish the genotype of Matsukawa (Kanto) from the genotypes of K326 and TN90. At a position corresponding to InDel3, insertion of bases "TT" is seen in Matsukawa (Kanto) and TN90, and insertion of a base "T" is seen in K326. Therefore, this marker can be used to distinguish the genotype of Matsukawa (Kanto) from the genotype of K326. At a position corresponding to InDel9, insertion of a base "T" is seen in Matsukawa (Kanto) and K326, and insertion of bases "TT" is seen in TN90. Therefore, this marker can be used to distinguish the genotype of Matsukawa (Kanto) from the genotype of TN90. Note that the reason why insertion of a base(s) is seen in K326 is that a difference is identified between sequence information of the foregoing reference genome used to specify the positions on Nt09 and the foregoing sequence data used to specify the genotypes of K326.

**[Table 4]**

| Marker ID | Insertion/deletion (compared with K326 reference genome) | | |
|---|---|---|---|
| | K326 | TN90 | Matsukawa (Kanto) |
| InDel1 | No insertion /deletion | No insertion /deletion | A insertion |
| InDel2 | No insertion /deletion | No insertion /deletion | AT insertion |
| InDel3 | T insertion | TT insertion | TT insertion |
| InDel4 | No insertion /deletion | No insertion /deletion | GAGA insertion |
| InDel5 | No insertion /deletion | T variation | Deletion |
| InDel6 | No insertion /deletion | No insertion /deletion | Deletion |
| InDel7 | A insertion | A insertion | AA insertion |
| InDel8 | TTTTT insertion | TTTTTT insertion | TTTTTTT insertion |
| InDel9 | T insertion | TT insertion | T insertion |
| InDel10 | No insertion /deletion | No insertion /deletion | Deletion |
| InDel11 | No insertion /deletion | Deletion | Deletion |
| InDel112 | No insertion /deletion | No insertion /deletion | Deletion |
| InDel13 | No insertion /deletion | Deletion | No insertion /deletion |
| InDel14 | T insertion | TT insertion | TT insertion |
| InDel15 | No insertion /deletion | T insertion | No insertion /deletion |
| InDel16 | No insertion /deletion | No insertion /deletion | T insertion |
| InDel17 | No insertion /deletion | A insertion | A insertion |
| InDel18 | No insertion /deletion | A insertion | A insertion |
| InDel19 | No insertion /deletion | No insertion /deletion | Deletion |
| InDel20 | No insertion /deletion | Deletion | No insertion /deletion |
| InDel21 | A insertion | A insertion | No insertion /deletion |

As described above, markers are also present each of which is identical to that in a genotype of the flue-cured variety or that in a genotype of the Burley variety. Therefore, the at least one marker used is at least one marker which is selected from the tables above and which can be distinguished from a genotype of the another variety crossed with Matsukawa (Kanto). For example, in a case where the flue-cured variety is used as the another variety which is crossed with Matsukawa (Kanto), at least one marker with use of which Matsukawa (Kanto) can be distinguished from the flue-cured variety may be selected and used to select the individual. In a case where the Burley variety is used as the another variety which is crossed with Matsukawa (Kanto), at least one marker with use of which Matsukawa (Kanto) can be distinguished from the Burley variety may be selected and used to select the individual. Alternatively, the genotype of each marker in the another variety which is crossed with Matsukawa (Kanto) may be identified in advance, and it may be determined whether the marker is appropriate as the at least one marker.

A method for identifying a marker only needs to allow determination of the presence or absence of a mutation in accordance with the type of the mutation to be the marker. Examples of the method encompass, but are not limited to, (1) a method in which a DNA sequence having the marker is amplified by PCR or the like, and then a DNA nucleotide sequence is directly decoded with use of a commercially available sequencer or the like, (2) a method in which a difference in sequence is detected by a difference in distance of electrophoresis by a single strand conformation polymorphism (SSCP) method, (3) a method in which single nucleotide polymorphism (SNP) is detected by a CycleavePCR method, (4) a method in which the presence or absence of the mutation is identified by cleaving a mismatch site(s) with use of T7 EndonucleaseI or the like, (5) a cleaved amplified polymorphic sequence (CAPS) method in which the presence or absence of the mutation can be determined by the presence or absence of cleavage by a restriction enzyme treatment, (6) a derived CAPS (dCAPS) method in which a set of primers including a mismatch intentionally is used so that the presence or absence of the mutation can be determined by the presence or absence of cleavage by restriction enzymes, (7) a method (e.g., a PCR method in which a TaqMan probe is used) in which the presence or absence of the mutation is determined by identifying, with use of a probe which specifically hybridizes to a mutant sequence, whether or not the probe is hybridized, (8) a method (MassARRAY analysis) in which a primer that is adjacent to the mutation is used to carry out single nucleotide extension so as to detect the presence or absence of the mutation by a difference in mass between nucleotides taken in, and (9) a method in which, in a case where the mutation is deletion or insertion, the mutation is identified by a difference in mobility of electrophoresis.

In the case of an InDel marker, examples of a preferably used method include, but are not limited to, a method in which a short PCR product containing the sequence of the InDel marker is amplified and a product containing insertion or deletion is electrophoretically distinguished from a product containing no insertion or deletion. Also in the case of an SNV marker, examples of a preferably used method include, but are not limited to, a method in which distinguishment is carried out by a restriction enzyme(s) and electrophoresis (CAPS method).

The number of markers used only needs to be 1 or more, and, for example, can be 1, 2, 3, 4, 5, 6, 7, 8, 9, or 10, or may be 11 or more.

The at least one marker used is also not particularly limited, but preferably includes any of InDel4, SNV1, and SNV95.

Examples of a method for confirming that the tobacco plant has a low nornicotine character encompass a nornicotine assay in which isatin staining is used. The assay includes a step of subjecting a seedling of the tobacco plant to a senescence treatment so as to promote conversion of nicotine to nornicotine, extracting the nornicotine from a part of a leaf and reacting the nornicotine with isatin, and determining the presence or absence of a nornicotine character in accordance with a degree of color development of a blue spot. As another method for confirming the low nornicotine character, a gas chromatography method (GC method) or a gas chromatography mass spectrometry method (GC-MS method) may be, for example, used.

### [Method for producing low reversion type low nornicotine content tobacco plant line]

With use of the foregoing selection method, a method for producing a low reversion type low nornicotine content tobacco plant line and a tobacco plant obtained by this production method are provided. The production method in accordance with an aspect of the present invention includes: a first step of crossing Matsukawa (Kanto), which is a variety of *Nicotiana tabacum,* and another variety of *Nicotiana tabacum* or a mutant of the another variety to obtain a first filial generation plant (hereinafter also referred to as F1); a second step of repeating backcrossing with respect to the first filial generation plant, the second step including (i) selecting, in each backcrossing, an individual having the at least one marker as a heterozygote and (ii) using the individual in next backcrossing; and a third step of selfing an individual obtained as a result of the second step to obtain a bred progeny, and carrying out the selection method to select an individual having the at least one marker as a homozygote.

The another variety of *Nicotiana tabacum* or the mutant of the another variety each of which is crossed with Matsukawa (Kanto) in the first step is an individual on which the low reversion type low nornicotine character of Matsukawa (Kanto) is desired to be conferred, and is not particularly limited, provided that the another variety or the mutant is one that can be crossed with Matsukawa (Kanto). The crossing and selection of the first filial generation plant only need to follow conventional methods. Since each first filial generation plant heterozygously has the genome structure of Matsukawa (Kanto) which is related to conferring of the low reversion type low nornicotine character, any first filial generation plant may be used in the second step.

In the second step, the backcrossing is carried out with respect to the first filial generation plant obtained in the first step. A cross parent used in the backcrossing is typically the same as that used in the crossing in the first step. Alternatively, the cross parent may be another *Nicotiana tabacum* variety. For example, in a case where a mutant obtained by an EMS treatment is used in the crossing in the first step, an individual which has the same background and which is not subjected to an EMS treatment can be used in the backcrossing without use of the mutant. Alternatively, in order to confer another excellent character, a variety which has another excellent character and which is grown for business use (so-called "elite variety") may be used in the backcrossing.

In the backcrossing, an individual which heterozygously has the genome structure of Matsukawa (Kanto) that is related to conferring of the low reversion type low nornicotine character and an individual which does not have the genome structure appear at a theoretical value of 1:1. Thus, in order to select the individual which has the genome structure of Matsukawa (Kanto) that is related to conferring of the low reversion type low nornicotine character, the at least one marker which is selected from Tables 1 and 2 and with use of which Matsukawa (Kanto) can be distinguished from the another variety used in the crossing in the first step and the variety used in the backcrossing in the second step is used to select, in each backcrossing, an individual which heterozygously has a genotype of Matsukawa (Kanto) in the at least one marker. Then, the individual which heterozygously has the genotype of Matsukawa (Kanto) in the at least one marker is used in the next backcrossing. In this manner, the backcrossing is repeated. The number of times of the backcrossing is not limited, and, for example, the backcrossing is carried out three to six times. Note that, for confirmation, measurement of a nornicotine content or identification of an NN-index described later may be carried out with respect to the individual which has been selected on the basis of a genotype of the at least one marker and which heterozygously has the genotype of Matsukawa (Kanto). Moreover, on the basis of the results of measurement of nornicotine contents or identification of NN-indexes described later, an individual which has a lower nornicotine content may be further selected from individuals each of which has been selected on the basis of the genotype of the at least one marker and each of which has the genotype of Matsukawa (Kanto).

In the third step, an individual which has been finally obtained in the second step, e.g., an individual which has been obtained by carrying out the backcrossing three to six times, is selfed, and an individual which homozygously has the genotype of Matsukawa (Kanto) in the at least one marker is selected. A method for selecting the individual which homozygously has the genotype of Matsukawa (Kanto) in the least one marker can be the foregoing method for selecting a low reversion type low nornicotine content tobacco plant. This makes it possible to produce a low reversion type low nornicotine content tobacco plant line derived from a tobacco plant having a desired background. Note that, for confirmation, measurement of a nornicotine content or identification of an NN-index described later may be carried out with respect to the tobacco plant selected on the basis of the genotype of the at least one marker. Moreover, on the basis of the results of measurement of nornicotine contents or identification of NN-indexes described later, an individual which has a lower nornicotine content may be further selected from the low reversion type low nornicotine content tobacco plant selected on the basis of the genotype of the at least one marker.

Namely, an embodiment of the present invention includes a low reversion type low nornicotine content tobacco plant obtained by the foregoing production method and an offspring of the low reversion type low nornicotine content tobacco plant. Therefore, each of these plants has the low reversion type low nornicotine character of Matsukawa (Kanto), although each of these plants is not Matsukawa (Kanto) itself. Each of these plants is characterized by having an endogenous CYP82E4 gene which encodes an amino acid represented by SEQ ID NO. 2 and having, as a homozygote, at least one marker selected from Tables 5 and 6 below. Markers shown in Tables 5 and 6 are each a marker with use of which Matsukawa (Kanto) can be distinguished from both the flue-cured variety K326 and the Burley variety TN90, among the markers shown in Tables 1 and 2.

**[Table 5]**

| Marker ID | Positions on Nt09 | Base | Marker ID | Positions on Nt09 | Base | Marker ID | Positions on Nt09 | Base |
|---|---|---|---|---|---|---|---|---|
| SNV2 | 101,505,318 | T | SNV30 | 101,698,257 | G | SNV60 | 101,875,101 | A |
| SNV4 | 101,523,017 | G | SNV31 | 101,718,066 | T | SNV61 | 101,378,001 | T |
| SNV5 | 101,524,311 | T | SNV32 | 101,721,614 | C | SNV67 | 101,903,020 | T |
| SNV7 | 101,526,729 | A | SNV33 | 101,725,448 | A | SNV69 | 101,908,076 | T |
| SNV8 | 101,544,000 | G | SNV34 | 101,725,867 | C | SNV70 | 101,910,271 | T |
| SNV9 | 101,545,638 | G | SNV35 | 101,727,812 | C | SNV74 | 101,918,202 | G |
| SNV10 | 101,550,197 | A | SNV36 | 101,737,537 | G | SNV75 | 101,918,533 | T |
| SNV11 | 101,562,465 | G | SNV37 | 101,737,694 | A | SNV76 | 101,925,101 | G |
| SNV12 | 101,569,477 | T | SNV39 | 101,742,957 | T | SNV77 | 101,933,909 | T |
| SNV15 | 101,579,509 | A | SNV40 | 101,744,875 | G | SNV78 | 101,952,245 | G |
| SNV16 | 101,582,099 | G | SNV41 | 101,748,913 | T | SNV79 | 101,968,528 | A |
| SNV17 | 101,583,478 | T | SNV44 | 101,754,559 | T | SNV80 | 101,976,846 | C |
| SNV13 | 101,584,177 | T | SNV45 | 101,777,902 | A | SNV82 | 101,989,567 | A |
| SNV19 | 101,586,295 | T | SNV47 | 101,786,341 | T | SNV84 | 101,992,887 | T |
| SNV20 | 101,586,769 | C | SNV49 | 101,303,382 | A | SNV85 | 101,992,988 | C |
| SNV22 | 101,614,452 | A | SNV50 | 101,822,302 | T | SNV86 | 102,013,873 | T |
| SNV23 | 101,636,959 | T | SNV51 | 101,825,828 | A | SNV87 | 102,016,365 | T |
| SNV24 | 101,637,377 | A | SNV52 | 101,826,725 | T | SNV83 | 102,018,158 | T |
| SNV25 | 101,640,243 | C | SNV53 | 101,838,779 | G | SNV92 | 102,053,413 | C |
| SNV27 | 101,651,143 | G | SNV56 | 101,363,951 | A | SNV93 | 102,061,071 | G |
| SNV23 | 101,669,996 | T | SNV59 | 101,374,929 | C | SNV94 | 102,067,002 | A |

**[Table 6]**

| Marker ID | Positions on Nt09 | Insertion/ deletion | Marker ID | Positions on Nt09 | Insertion/ deletion |
|---|---|---|---|---|---|
| InDel1 | 101544774^101544775 | A insertion | InDel8 | 101870399^101870400 | TTTTTTT insertion |
| InDel2 | 101561948^101561949 | AT insertion | InDel10 | 101,331,218 | Deletion |
| InDel4 | 101685327^101685328 | GAGA insertion | InDel12 | 101,897,361 | Deletion |
| InDel5 | 101,822,299 | Deletion | InDel16 | 101953185^101953186 | T insertion |
| InDel6 | 101,855,430 | Deletion | InDel19 | 102040788..102040807 | Deletion |
| InDel7 | 101867656^101867657 | AA insertion | InDel21 | 102073969^102073970 | No insertion /deletion |

Aspects of the present invention can also be expressed as follows:
With the above embodiments considered together, the present invention can be summarized as follows.

(1) A method for selecting a low reversion type low nornicotine content tobacco plant, including
   a step of, from a bred progeny line obtained by crossing Matsukawa (Kanto), which is a variety of *Nicotiana tabacum,* and another variety of *Nicotiana tabacum* or a mutant of the another variety, selecting an individual having, as a homozygote, at least one marker which is of Matsukawa (Kanto), which is selected from Tables 1 and 2, and which allows Matsukawa (Kanto) to be distinguished from the another variety or the mutant of the another variety.
(2) The method described in (1), wherein the another variety is a Burley variety or a flue-cured variety.
(3) The method described in (1) or (2), wherein the at least one marker includes at least any one of InDel4, SNV1, and SNV95.
(4) The method described in (1) or (2), wherein the at least one marker includes at least InDel4.
(5) A method for producing a low reversion type low nornicotine content tobacco plant line, including:
   a first step of crossing Matsukawa (Kanto), which is a variety of *Nicotiana tabacum,* and another variety of *Nicotiana tabacum* or a mutant of the another variety to obtain a first filial generation plant;
   a second step of repeating backcrossing with respect to the first filial generation plant with use of *Nicotiana tabacum* other than Matsukawa (Kanto) as a backcross parent, the second step including (i) selecting, in each backcrossing, an individual having, as a heterozygote, at least one marker which is of Matsukawa (Kanto), which is selected from tables above, and which allows Matsukawa (Kanto) to be distinguished from the another variety or the mutant and the backcross parent and (ii) using the individual in next backcrossing; and
   a third step of selfing an individual obtained as a result of the second step to obtain a bred progeny, and carrying out a method described in (1) to select an individual having the at least one marker as a homozygote.
(6) A low reversion type low nornicotine content tobacco plant obtained by a method described in (5).
(7) A low reversion type low nornicotine content tobacco plant (other than Matsukawa (Kanto), which is a variety of *Nicotiana tabacum*), including:
   an endogenous CYP82E4 gene which encodes an amino acid sequence represented by SEQ ID NO. 2; and
   as a homozygote, at least one marker which is selected from Tables 5 and 6.

The following description will more specifically discuss embodiments of the present invention with reference to Examples. The present invention is, of course, not limited to Examples below and particulars can have various aspects. Further, the present invention is not limited to the embodiments, but can be altered by a skilled person in the art within the scope of the claims. An embodiment derived from a proper combination of technical means disclosed in respective different embodiments is also encompassed in the technical scope of the present invention. Moreover, all the literatures described herein are hereby incorporated by reference.

### Examples

### (1) Tobacco plant

As varieties in the present example, used were (i) Matsukawa (Kanto), which is a Japanese local variety, (ii) TN90 and Burley 21 (No. 3-10), each of which is a Burley variety, and (iii) K326, Tsukuba 1, and a Tsukuba 1 mutant, each of which is a flue-cured variety. Each of TN90, K326, and Tsukuba 1 is low converter type tobacco having a low conversion rate of nicotine to nornicotine. Burley 21 (No. 3-10) is high converter type tobacco having a high conversion rate of nicotine to nornicotine. Moreover, an F2 population prepared by selfing F1 seeds obtained by crossing Matsukawa (Kanto) and Burley 21 (No. 3-10) was used for analyses. Plants were grown in a greenhouse.

### (2) Low reversion type low nornicotine character of Matsukawa (Kanto)

In the greenhouse, 226 individuals of Matsukawa (Kanto) were grown, and nornicotine assays were carried out. In the nornicotine assays, used was a method in which induction of senescence of a seedling (Shi et al. 2003, J. Agric. Food Chem. 51: 7679-7683.) and isatin staining (Sato and Asaina, 1982, Bulletin of the Iwata Tobacco Experiment Station 14: 17-21.) were combined. Fully expanded leaves of seedlings which were grown in the greenhouse and which were 6 to 7 weeks after sowing (the seedlings obtained in the 3rd to 4th week after the seedlings which were 3 weeks after the sowing were temporarily planted in 4×4 vinyl pots) were treated at 37°C and a humidity of 85% for 4 days (senescence treatment) so that conversion of nicotine to nornicotine was promoted. After that, parts (tip parts each measuring approximately 1.5 cm²) of the leaves were put in 1.5-mL Eppendorf tubes. The parts were immersed in 250 µL of a strongly alkaline solution (8M NaOH) containing 1% of surfactant Tween-20 so that cells were crushed. Thereafter, 500 µL of chloroform was added. A resulting solution was left to stand still for 1 hour so that nornicotine was extracted. On filter paper (Absorbent paper CB-09A, manufactured by ATTO CORPORATION), 20 µL of a chloroform layer, which was a lower layer, was spotted. After the dropped solution was dried, an isatin reagent [obtained by dissolving 0.1 g of 2,3-Indolinedione (manufactured by FUJIFILM Wako Pure Chemical Corporation) in 2.5 mL of acetic acid and 50 mL of ethanol] was uniformly sprayed on the filter paper with use of a glass sprayer. The obtained filter paper was treated at 120°C for 4 minutes in a warm air dryer. Nornicotine exhibits color development of a blue spot as a result of a reaction between isatin and the nornicotine. As indexes of the color development, an indicator which had five levels of indexes (NN-index = 1 to 5) corresponding to degrees of the color development was prepared by dropping standard nornicotine in the same manner. Specifically, an indicator which was divided into five levels of standard nornicotine contents of 0.05%, 0.1%, 0.25%, 0.5%, and 1% was prepared as the indexes of the color development, and referred to for the degrees of the color development. These indexes of the color development were respectively regarded as nornicotine index values (or nornicotine indexes) 1, 2, 3, 4, and 5. With use of this indicator, NN-indexes were determined from the degrees of the color development. In this indicator, a higher NN-index indicates that more nornicotine is contained.

The results of nornicotine assays of each line are shown in Table 7. In Burley 21 (No. 3-10), which served as a control, all 23 individuals exhibited an NN-index of 3 or more, and nearly 70% of the individuals exhibited an NN-index of 4 or 5. That is, all the individuals of Burley 21 (No. 3-10) had high nornicotine contents, and were of a typical high converter type. In TN90, 30 out of 48 individuals exhibited an NN-index of 1 or 2, that is, had low nornicotine contents, but 9 individuals exhibited an NN-index of 4 or 5, that is, appearance of high nornicotine type converters was seen. In contrast, in Matsukawa (Kanto), all 226 individuals exhibited an NN-index of 1 or 2, that is, had low nornicotine contents, and an individual exhibiting an NN-index of 3 or more was not seen. Thus, it was confirmed that Matsukawa (Kanto) was a tobacco variety in which a high nornicotine type converter did not appear or was extremely unlikely to appear.

**[Table 7]**

| Lines | NN-index | | | | | Number of individuals analyzed |
|---|---|---|---|---|---|---|
| | 1 | 2 | 3 | 4 | 5 | |
| Matsukawa (Kanto) | 154 | 72 | 0 | 0 | 0 | 226 |
| Burley 21 (No.3-10) | 0 | 0 | 5 | 11 | 7 | 23 |
| TN90 | 17 | 13 | 9 | 6 | 3 | 48 |

### (3) Mutation in CYP82E4 gene of Matsukawa (Kanto)

Next, with reference to the publicly known genome base sequence of a CYP82E4 gene of TN90 (SEQ ID NO. 4 in Japanese Patent Application Publication Tokukai No. 2008-506362, and GenBank accession No.: AYMY01253998), the base sequence of a CYP82E4 gene of Matsukawa (Kanto) was determined, and compared with the sequences of the same genes of TN90, Burley 21 (No. 3-10), and K326. A genomic DNA was extracted from a leaf of a seedling of Matsukawa (Kanto) with use of Gentra Puregene Tissue Kit (manufactured by Qiagen). With use of this DNA as a template, each of the first half (PCR1) and the second half (PCR2) of the CYP82E4 gene (approximately 6,500 bp) was amplified by PCR, and sequences were determined. The PCR was carried out under the following conditions: 5 ng of the template DNA and 5 pmol of each of primers shown in Table 8 were added to 20 µL of a reaction solution; Tks(GFlex)DNA Polymerase (manufactured Takara Bio Inc.) was used; and 40 rounds of PCR were carried out in each of which PCR was carried out at 96°C for 10 seconds, at 55°C for 10 seconds, and then at 72°C for 15 seconds. PCR products (a product of approximately 3.1 kb from PCR1 and a product of 3.4 kb from PCR2) were treated with use of ExoSAP-IT (manufactured by Thermo Fisher) to remove the PCR primers. After that, the base sequences were determined with use of a fluorescent sequencer. Furthermore, the whole base sequence of the CYP82E4 gene was determined by a primer walking method.

**[Table 8]**

| **Target** | **Primer** | **Sequence (5' to 3' )** | **Length** | **SEQ ID NO.** |
|---|---|---|---|---|
| PCR1 (**Approx**. 3.1 kb) | Forward primer 1 | ctcggattcagctatcaaagg | 21 mer | 3 |
| | Reverse primer 2 | catccagcatccagcattgc | 20 mer | 4 |
| PCR2 (**Approx**. 3 4kb) | Forward primer 1 | gcatcatacccctaattggag | 21 mer | 5 |
| | Reverse primer 2 | cttctgcggagtcgtatctg | 20 mer | 6 |

As a result, in TN90, which was a control, 12 repeats of GA were present in the promoter region located approximately 500 bp upstream of the translation initiation codon ATG of the CYP82E4 gene, whereas, in Matsukawa (Kanto), insertion of four bases "GAGA" (hereafter also referred to as "GAGA insertion") was found. In other words, 14 repeats of GA were present in the promoter of the CYP82E4 gene of Matsukawa (Kanto). No mutation was found in the other regions. On the other hand, in Burley 21 (No. 3-10), as a result of carrying out the same experiment as that described above, 12 repeats of GA were present. When the genome sequence of CYP82E4 of K326, which is published in a publicly known database, was searched, 12 repeats of GA were present. Note that, also in Tsukuba 1, 12 repeats of GA were present. Moreover, as a result of carrying out determination regarding Matsukawa (Fukushima), 13 repeats of GA were present in Matsukawa (Fukushima). Thus, it was confirmed that Matsukawa (Fukushima) was distinguished from Matsukawa (Kanto) in accordance with a sequence on a genome.

### (4) Obtainment of mutant of CYP82E4 gene (experiment control)

As an experiment control, a tobacco mutant in which a CYP82E4 gene was disrupted was obtained. From the Tsukuba 1 mutant library (Tajima et al., 2011, Ann. Phytopathol. Soc. Jpn. 77:258., Takakura et al., 2018, Mol. Plant Pathol. 19 2124-2133.), a mutant having a nonsense mutation in the first exon of a CYP82E4 gene (SEQ ID NO. 4 in Japanese Patent Application Publication Tokukai No. 2008-506362) (a mutant in which the 2798th base of SEQ ID NO. 1 in the sequence list was substituted from G to A, and, as a result, a codon TGG was altered to TGA) was selected. After an amplification product was obtained with use of the primers (for PCR1) shown in Table 8 above, a base sequence was determined with use of primers for mutation detection (Table 9), and a line in which the mutation was homozygous was obtained. The obtained mutant line was designated as e4-TUM.

**[Table 9]**

| **Primer** | **Sequence (5' to 3' )** | **SEQ ID NO.** |
|---|---|---|
| e4-FW1 | ATCGTGAAGATGATCGCT | 7 |
| e4-RV1 | AGGCTCGAACCTGTACAC | 8 |

### (5) Determination of mode of inheritance of low reversion type low nornicotine character of Matsukawa (Kanto) and linkage with GAGA insertion

As has been described above, it was shown that, in Matsukawa (Kanto), a high nornicotine type converter did not appear or was extremely unlikely to appear. In order to investigate whether a genetic factor that brings about this low reversion type low nornicotine character of Matsukawa produces its effect even in a case where Matsukawa (Kanto) is crossed with another variety and whether a low nornicotine individual can be selected with use of the GAGA insertion as an index, the F2 population of the cross progeny of Matsukawa (Kanto) and Burley 21 (No. 3-10) was used to analyze linkage between the GAGA insertion and a nornicotine type. A method for determining the presence or absence of the GAGA insertion was as follows. That is, PCR in which, as described above, DNA extracted from a leaf of a seedling in the 7th week from sowing was used as a template and the same primers (Forward primer 1 and Reverse primer 1) as those in TargetPCR1 shown in Table 8 were used was similarly carried out, and the base sequence of an amplification product was determined with use of primers for sequencing near the GAGA insertion that are shown in Table 10. It was determined, with use of the waveform of sequence data, which one of a homozygote, a heterozygote, and a null the GAGA insertion was.

**[Table 10]**

| **Primer** | **Sequence (5' to 3' )** | **Length** | **SEQ ID NO.** |
|---|---|---|---|
| GAGA-seq1 | aggatttgaaacttatgggtt | 21 mer | 9 |
| GAGA-seq2 | gttacgtatggagtttgaacc | 21 mer | 100 |

Table 11 shows the results of nornicotine assays of the F2 population of the cross progeny of Matsukawa (Kanto) and Burley 21 (No. 3-10) and control varieties. Out of 49 individuals each homozygously having the GAGA insertion, 47 individuals exhibited an NN-index of 1, and 2 individuals exhibited an NN-index of 2. No individual exhibited an NN-index of 3 or more. The same was true of the results for the tobacco mutant line (e4-TUM) which was a control and which had the nonsense mutation in the CYP82E4 gene. Meanwhile, in a population which heterozygously had the GAGA insertion or did not have the GAGA insertion, individuals exhibiting an NN-index of 1 or 2 were also seen sporadically, but most individuals exhibited an NN-index of 3 or more. In TN90 and Tsukuba 1 (both of which were subjected to seed selection) each of which was a control, many individuals exhibited an NN-index of 1 or 2. However, 2 out of 47 individuals in TN90 and 6 out of 48 individuals in Tsukuba 1 exhibited an NN-index of 4 or more. Thus, appearance of high nornicotine type converters was seen in TN90 and Tsukuba 1. The manner of the appearance of the high nornicotine type converters in TN90 (appearance of 2 out of 47 individuals) was similar to that of appearance in TN90LC of Verrier et al. (2011) (CORESTA Meeting AP06). The manner of the appearance of the high nornicotine type converters in Tsukuba 1 (appearance of 6 out of 47 individuals) was similar to that of appearance in Kubo (1985) (Bulletin of the Iwata Tobacco Experiment Station 17: 69-137). From the above facts, it was clarified that the GAGA insertion in Matsukawa (Kanto) linked with the low reversion type low nornicotine character and a low reversion type low nornicotine content tobacco plant was able to be selected with use of the GAGA insertion as an index.

**[Table 11]**

| Lines | | NN-index | | | | | Number of individuals analyzed |
|---|---|---|---|---|---|---|---|
| | | 1 | 2 | 3 | 4 | 5 | |
| F2-GAGA insertion null | | 1 | 1 | 6 | 26 | 10 | 44 |
| F2-GAGA insertion hetero | | 4 | 3 | 31 | 37 | 19 | 94 |
| F2-GAGA insertion homo | | 47 | 2 | 0 | 0 | 0 | 49 |
| | Subtotal | 52 | 6 | 37 | 63 | 29 | 187 |
| Matsukawa(Kanto) | | 10 | 0 | 0 | 0 | 0 | 10 |
| Burley 21 (No. 3-10) | | 1 | 0 | 2 | 6 | 1 | 10 |
| TN90 | | 25 | 13 | 7 | 1 | 1 | 47 |
| e4-TUM | | 47 | 1 | 0 | 0 | 0 | 48 |
| Tsukuba 1 | | 36 | 2 | 4 | 5 | 1 | 48 |

As a cause of appearance of an NN-index of 1 or 2 in the individuals of the F2 population in each of which the GAGA insertion was heterozygous or null (no GAGA insertion), it was also considered that a small amount of nicotine, which is a substrate of nornicotine, accumulated. Therefore, all 58 individuals in the F2 population each of which exhibited an NN-index of 1 or 2 and, as a control, 5 individuals in TN90 each of which exhibited an NN-index of 1, in the results shown in Table 11, were further transplanted from the vinyl pots into 12-cm pots, and biosynthesis of nicotine was promoted. Then, nornicotine assays were further carried out 3 weeks later. In the individuals in each of which the GAGA insertion was heterozygous or null at a time point when 4 weeks elapsed from temporary transplantation, all the individuals each of which exhibited an NN-index of 1 or 2 came to exhibit an NN-index of 4 or 5 after 3 weeks elapsed from the transplantation. In the individuals in each of which the GAGA insertion was homozygous, all the individuals each of which exhibited an NN-index of 1 or 2 remained exhibiting an NN-index of 1 or 2. In this manner, all the individuals each of which homozygously had the GAGA insertion came to have low nornicotine contents, and no high nornicotine type converter appeared. In contrast, out of the five individuals in TN90 each of which exhibited an NN-index of 1, one individual remained exhibiting an NN-index of 1, two individuals come to exhibit an NN-index of 2, and two individuals came to exhibit an NN-index of 3. In this manner, the NN-index gradually increased with growth. Table 12 shows results obtained by combining these results and Table 11. In the F2 population, the number of the individuals each of which exhibited an NN-index of 1 or 2 (no appearance of a high nornicotine type converter) was 49, and the number of the individuals each of which exhibited an NN-index of 3 or more was 138, which agreed well with a 1:3 segregation ratio (P = 0.704, χ2 test). This clarified that the mode of inheritance of the low reversion type low nornicotine character of Matsukawa (Kanto) was single-factor recessive.

**[Table 12]**

| Lines | | NN-index | | | | | Number of individuals analyzed |
|---|---|---|---|---|---|---|---|
| | | 1 | 2 | 3 | 4 | 5 | |
| F2-GAGA insertion null | | 0 | 0 | 6 | 26 | 12 | 44 |
| F2-GAGA insertion hetero | | 0 | 0 | 31 | 40 | 23 | 94 |
| F2-GAGA insertion homo | | 36 | 13 | 0 | 0 | 0 | 49 |
| | Subtotal | 36 | 13 | 37 | 66 | 35 | 187 |

Thus, it was clarified that a high nornicotine type converter did not appear or was extremely unlikely to appear when GAGA insertion was homozygously present and that a low reversion type low nornicotine content tobacco plant was able to be selected with use of the GAGA insertion as an index.

### (6) Genome analysis of Matsukawa (Kanto), detection of polymorphisms other than GAGA insertion, and application thereof to selection

By analyzing the whole genome sequence of Matsukawa (Kanto) and comparing the genome sequence with the genome sequences of K326, which is a representative flue-cured variety, and TN90, which is a representative Burley variety, polymorphisms which were present near the GAGA insertion and which were seen between Matsukawa (Kanto) and the flue-cured variety and/or the Burley variety (SNV [single nucleotide variation] and short Indel [insertion, deletion]) were detected. Genome sequence information of Matsukawa (Kanto) was obtained by sequencing, with use of a DNB-SEQ^{™} (manufactured by BGI), DNA extracted from a leaf of a seedling. As genome sequence information of TN90, published sequence data (accession no. SRR954964 in the Sequence Read Archive (SRA) of GenBank) was used. Moreover, as genome sequence information of K326, published K326 reference genome information ((ftp://ftp.solgenomics.net/genomes/Nicotiana_tabacum/edwards_et_al_2017/assembly/Nitab-v4.5_genome_Chr_Edwards2017.fasta) and published sequence data (accession no. SRR955769 in the Sequence Read Archive (SRA) of GenBank) were used. As a result, as markers with use of which Matsukawa (Kanto) could be distinguished from K326 or TN90, SNV1 to SNV95 shown in Table 1 above and InDel1 to InDel21 shown in Table 2 above were detected. Note that the GAGA insertion corresponds to InDel4 among these markers.

Among the markers shown in Tables 1 and 2, SNV1 and SNV95, which are the two most distant on the genome sequence, were used to carry out analyses. SNV1 is single nucleotide variation which is on an NLP7 gene and which is located approximately 192 kb upstream from the GAGA insertion. SNV95 is single nucleotide variation which is on a ZF gene and which is located approximately 398 kb downstream from the GAGA insertion. DNA markers for detecting SNV1 and SNV95 were developed (Table 13: Forward primer and Reverse primer are for PCR, and Sequencing primers are for sequencing). Subsequently, genotyping analyses were carried out with use of DNA samples from F2 of Matsukawa (Kanto) and Burley 21 (No. 3-10). The types of the GAGA insertion and the results of NN-indexes were compared. PCR was carried out once at 94°C for 2 minutes, carried out 40 times at 98°C for 10 seconds, 55°C for 15 seconds, and 68°C for 5 seconds, and then carried out once at 68°C for 5 minutes. The PCR was carried out with use of KOD One (manufactured by Toyobo Co., Ltd.). The base sequences of PCR products were determined by Sanger sequencing, and analyzed by the sequence assembly software ATGC (manufactured by GENETYX). Results are shown in Tables 14 and 15.

**[Table 13]**

| **Target gene** | **Primer** | **Sequence (5' to 3' )** | **SEQ ID NO.** |
|---|---|---|---|
| NLP7 | Forward | GAACAATTATGGGAAATTGTACTCTC | 11 |
| | Reverse | GCAAAGTTTCTTGAGCAAGC | 12 |
| | Sequencing | CAAGAGAAGCTTCATTGATAGAC | 13 |
| ZF | Forward | AGAAACTTCCATCAAGATCAAC | 14 |
| | Reverse | CTACTACAAGCAAATGACTGAA | 15 |
| | Sequencing | CAGGGACCATTTTCTATGGTC | 16 |

As shown in Table 14, among individuals in each of which the GAGA insertion (InDel4) was homozygous, 48 individuals in each of which data on SNV1 was obtained all homozygously had a genotype of Matsukawa (Kanto) at SNV1. All these individuals exhibited an NN index of 1 or 2. Meanwhile, one individual was seen which homozygously had the genotype of Matsukawa (Kanto) at SNV1 but heterozygously had the GAGA insertion (InDel4) and which exhibited an NN-index of 4. This clarified that a low nornicotine type was able to be selected with a probability of 48/49 = 98% when an individual having the genotype of Matsukawa (Kanto) was selected with use of the SNV1 marker as an index.

**[Table 14]**

| GAGA Matsukawa (Kanto) type insertion | NLP Matsukawa (Kanto) type base | Number of samples | NN index | | | | |
|---|---|---|---|---|---|---|---|
| | | | 1 | 2 | 3 | 4 | 5 |
| homo | homo | 48 | 46 | 2 | 0 | 0 | 0 |
| homo | - | 1 | 1 | 0 | 0 | 0 | 0 |
| hetero | homo | 1 | 0 | 0 | 0 | 1 | 0 |
| hetero | hetero | 86 | 0 | 0 | 30 | 35 | 21 |
| hetero | null | 1 | 0 | 0 | 1 | 0 | 0 |
| hetero | - | 1 | 0 | 0 | 0 | 0 | 1 |
| null | null | 47 | 0 | 0 | 10 | 24 | 13 |
| null | - | 2 | 0 | 0 | 1 | 1 | 0 |

| | | | | | | | |
|---|---|---|---|---|---|---|---|
| ("-" indicates that the re is no data..) | | | | | | | |

Moreover, as shown in Table 15, among individuals in each of which the GAGA insertion (InDel4) was homozygous, 47 out of 48 individuals in each of which data on SNV95 was obtained homozygously had a genotype of Matsukawa (Kanto) at SNV95, and 1 out of 48 individuals heterozygously had the genotype of Matsukawa (Kanto) at SNV95. All these individuals exhibited an NN index of 1 or 2. Meanwhile, one individual was seen which homozygously had the genotype of Matsukawa (Kanto) in SNV95 but heterozygously had the GAGA insertion (InDel4) and which exhibited an NN-index of 4. This clarified that a low nornicotine type was able to be selected with a probability of 47/48 = 98% when an individual having the genotype of Matsukawa (Kanto) was selected with use of the SNV95 marker as an index. The probability of missing an individual originally exhibiting an NN-index of 1 was 1/48 = 2%.

**[Table 15]**

| GAGA Matsukawa (Kanto) type insertion | ZF Matsukawa (Kanto) type base | | NN index | | | | |
|---|---|---|---|---|---|---|---|
| | | | 1 | 2 | 3 | 4 | 5 |
| homo | homo | 47 | 45 | 2 | 0 | 0 | 0 |
| homo | hetero | 1 | 1 | 0 | 0 | 0 | 0 |
| homo | - | 1 | 1 | 0 | 0 | 0 | 0 |
| hetero | homo | 1 | 0 | 0 | 0 | 1 | 0 |
| hetero | hetero | 85 | 0 | 0 | 30 | 33 | 22 |
| hetero | null | 2 | 0 | 0 | 0 | 2 | 0 |
| hetero | - | 1 | 0 | 0 | 1 | 0 | 0 |
| null | hetero | 2 | 0 | 0 | 0 | 1 | 1 |
| null | null | 44 | 0 | 0 | 11 | 22 | 11 |
| null | - | 3 | 0 | 0 | 0 | 2 | 1 |

| | | | | | | | |
|---|---|---|---|---|---|---|---|
| ("-" indicates that there is no data.) | | | | | | | |

Thus, it was shown that both the SNV1 and SNV95 markers made it possible to, with a high probability, select a low reversion type low nornicotine content tobacco plant.

### (7) Analysis of nornicotine ratios of cross progeny (BC5F2) line of Matsukawa (Kanto) and TN90

Backcrossing was carried out with use of Matsukawa (Kanto) as a nonrecurrent parent and TN90 as a backcross parent (recurrent parent). F1 was obtained by crossing pollen of Matsukawa (Kanto) with TN90 which was a seed parent. Furthermore, F1 was crossed with pollen of TN90 which was a backcross parent. In theory, an individual which heterozygously has both a GAGA insertion mutation and single nucleotide variation in NLP7 that are derived from Matsukawa (Kanto) and an individual which does not have both the mutations appear at a segregation ratio of 1:1. Among these individuals, a heterozygous individual was selected with use of the GAGA insertion mutation (Indel4) and the single nucleotide variation (SNV1) in NLP7 as indexes. Determination of the presence or absence of the GAGA insertion was carried out as follows. That is, PCR in which, as described above, DNA extracted from a leaf of a seedling was used as a template and the same primers (Forward primer 1 and Reverse primer 1) as those in TargetPCR1 shown in Table 8 were used was similarly carried out, and the base sequence of an amplification product was determined with use of primers for sequencing near the GAGA insertion that are shown in Table 10. The single nucleotide variation in NLP7 was determined as follows. That is, PCR in which, as described above, the same Forward and Reverse primers for NLP7 as those shown in Table 13 above were used was similarly carried out, and the base sequence of an amplification product was determined with use of a primer for sequencing near the single nucleotide variation in NLP7 that is shown in Table 13. A selected individual was further crossed with TN90, and the above operation was repeated. A BC5F2 generation which was obtained by selfing a BC5F1 generation obtained by carrying out crossing a total of five times with use of TN90 as a pollen parent was subjected to a field experiment. In the BC5F2 generation, individuals each of which homozygously had both the GAGA insertion mutation and the single nucleotide variation in NLP7 and individuals in each of which the GAGA insertion mutation and the single nucleotide variation in NLP7 were both null (having none of these mutations) were transplanted to a field after genotype selection was carried out in the same manner as described above when these individuals were seedlings. Note that, in all the individuals in which the GAGA insertion mutation was homozygous, the single nucleotide variation in NLP7 was also homozygous. In all the individuals in which the GAGA insertion mutation was null, the single nucleotide variation in NLP7 was also null. The plants were grown in the field, topping was carried out during flowering time, 3 leaves from the top were sampled for each individual 50 days after the topping, and the samples were naturally cured for 37 days. The cured samples were ground, and then nicotine and nornicotine were analyzed by a GC-MS method. The ratio of a nornicotine content to the total value of a nicotine content and the nornicotine content was shown as a nornicotine ratio. The results are shown in Fig. 1. Note that, for convenience, individual numbers are given in ascending order from an individual having a low nornicotine ratio. The appearance rate of an individual having a nornicotine ratio of 5% or more was as follows: 7 (4.0%) out of 174 individuals in a line in which the GAGA insertion mutation was homozygous; 44 (38.3%) out of 115 individuals in a line in which the GAGA insertion mutation was null. The appearance rate of an individual having a nornicotine ratio of 10% or more was as follows: 1 (0.6%) out of 174 individuals in the line in which the GAGA insertion mutation was homozygous; 14 (12.2%) out of 115 individuals in the line in which the GAGA insertion mutation was null. Thus, it was shown that a low reversion type low nornicotine individual was able to be selected also in a cross progeny line with use of the GAGA insertion mutation (Indel4) or the single nucleotide variation (SNV1) in NLP7, each of which was derived from Matsukawa (Kanto), as indexes.

### Industrial Applicability

The present invention can be used to produce a variety or a line of *Nicotiana tabacum.*

## Claims

1. A method for selecting a low reversion type low nornicotine content tobacco plant, comprising
a step of, from a bred progeny line obtained by crossing Matsukawa (Kanto), which is a variety of *Nicotiana tabacum,* and another variety of *Nicotiana tabacum* or a mutant of the another variety, selecting an individual having, as a homozygote, at least one marker which is of Matsukawa (Kanto), which is selected from tables below, and which allows Matsukawa (Kanto) to be distinguished from the another variety or the mutant of the another variety, wherein
markers indicated by SNV are each a marker based on single nucleotide variation present at a position corresponding to each shown position on a chromosome Nt09 in a reference genome sequence of K326 of *Nicotiana tabacum,*
a "Base" indicates a base in Matsukawa (Kanto),
markers indicated by InDel are each a marker based on insertion or deletion present at a position corresponding to each shown position on the chromosome Nt09 in the reference genome sequence, and
"Insertion/Deletion" indicates whether the insertion or the deletion is present or not in Matsukawa (Kanto).
**[Table 1]**
| Marker **ID** | Positions on Nt09 | Base | Marker ID | Positions on Nt09 | Base | Marker ID | Positions on Nt09 | Base |
|---|---|---|---|---|---|---|---|---|
| SNV1 | 101,496,032 | C | SNV33 | 101,725,448 | A | SNV65 | 101,893,439 | T |
| SNV2 | 101,505,318 | T | SNV34 | 101,725,867 | C | SNV66 | 101,902,591 | C |
| SNV3 | 101,508,443 | C | SNV35 | 101,727,812 | C | SNV67 | 101,903,020 | T |
| SNV4 | 101,523,017 | G | SNV36 | 101,737,537 | G | SNV68 | 101,906,181 | G |
| SNV5 | 101,524,311 | T | SNV37 | 101,737,694 | A | SNV69 | 101,908,076 | T |
| SNV6 | 101,526,270 | G | SNV33 | 101,740,101 | C | SNV70 | 101,910,271 | T |
| SNV7 | 101,526,729 | A | SNV39 | 101,742,957 | T | SNV71 | 101,914,414 | A |
| SNV8 | 101,544,000 | G | SNV40 | 101,744,875 | G | SNV72 | 101,916,403 | A |
| SNV9 | 101,545,638 | G | SNV41 | 101,748,913 | T | SNV73 | 101,917,044 | A |
| SNV10 | 101,550197 | A | SNV42 | 101,751,457 | T | SNV74 | 101,918,202 | G |
| SNV11 | 101,562,465 | G | SNV43 | 101,752,576 | G | SNV75 | 101,918,533 | T |
| SNV12 | 101,569,477 | T | SNV44 | 101,754,559 | T | SNV76 | 101,925,101 | G |
| SNV13 | 101,572,171 | C | SNV45 | 101,777,902 | A | SNV77 | 101,933,909 | T |
| SNV14 | 101,578,805 | C | SNV46 | 101,786,139 | G | SNV78 | 101,952,245 | G |
| SNV15 | 101,579,509 | A | SNV47 | 101,786,341 | T | SNV79 | 101,968,528 | A |
| SNV16 | 101,582,099 | G | SNV48 | 101,787,252 | C | SNV80 | 101,976,846 | C |
| SNV17 | 101,583,478 | T | SNV49 | 101,303,382 | A | SNV81 | 101,988,964 | T |
| SNV18 | 101,584,177 | T | SNV50 | 101,822,302 | T | SNV82 | 101,989,567 | A |
| SNV19 | 101,586,295 | T | SNV51 | 101,825,828 | A | SNV83 | 101,989,748 | G |
| SNV20 | 101,586,769 | C | SNV52 | 101,826,725 | T | SNV84 | 101,992,887 | T |
| SNV21 | 101,586,820 | A | SNV53 | 101,838,779 | G | SNV35 | 101,992,988 | C |
| SNV22 | 101,614,452 | A | SNV54 | 101,843,000 | C | SNV86 | 102,013,873 | T |
| SNV23 | 101,636,959 | T | SNV55 | 101,859,983 | C | SNV87 | 102,016,365 | T |
| SNV24 | 101,637,377 | A | SNV56 | 101,863,951 | A | SNV88 | 102,018,158 | T |
| SNV25 | 101,640,243 | C | SNV57 | 101,367,595 | C | SNV89 | 102,037,650 | A |
| SNV26 | 101,643,526 | C | SNV58 | 101,870,376 | C | SNV90 | 102,039,954 | G |
| SNV27 | 101,651,143 | G | SNV59 | 101,874,929 | C | SNV91 | 102,041,266 | A |
| SNV28 | 101,669,996 | T | SNV60 | 101,875,101 | A | SNV92 | 102,053,413 | C |
| SNV29 | 101,697,595 | T | SNV61 | 101,878,001 | T | SNV93 | 102,061,071 | G |
| SNV30 | 101,698,257 | G | SNV62 | 101,886,773 | G | SNV94 | 102,067,002 | A |
| SNV31 | 101,718,066 | T | SNV63 | 101,892,276 | A | SNV95 | 102,089,629 | A |
| SNV32 | 101,721,614 | C | SNV64 | 101,893,204 | C | | | |
**[Table 2]**
| Marker ID | Positions on Nt09 | Insertion/ deletion | Marker ID | Positions on Nt09 | Insertion/ deletion |
|---|---|---|---|---|---|
| InDel1 | 101544774^101544775 | A insertion | InDel12 | 101,897,361 | Deletion |
| InDel2 | 101561948^101561949 | AT insertion | InDel13 | 101,916,401 | No insertion /deletion |
| InDel3 | 101564173^101564174 | TT insertion | InDel14 | 101925961^101925962 | TT insertion |
| InDel4 | 101685327^101685328 | GAGA insertion | InDel15 | 101953087^101953088 | No insertion /deletion |
| InDel15 | 101,822,299 | Deletion | InDel16 | 101953185^101953186 | T insertion |
| InDel6 | 101,855,430 | Deletion | InDel17 | 101974552^101974553 | A insertion |
| InDel7 | 101867656^101867657 | AA insertion | InDel18 | 102003223^102003224 | A insertion |
| InDel8 | 101870399^101870400 | TTTTTTT insertion | InDel19 | 102040788..102040301 | Deletion |
| InDel9 | 101873449^101873450 | T insertion | InDel20 | 102,048,990 | No insertion /deletion |
| InDel10 | 101,881,218 | Deletion | InDel21 | 102073969^102073970 | No insertion /deletion |
| InDel11 | 101,884,256 | Deletion | | | |

2. The method as set forth in claim 1, wherein the another variety is a Burley variety or a flue-cured variety.

3. The method as set forth in claim 1 or 2, wherein the at least one marker includes at least any one of InDel4, SNV1, and SNV95.

4. The method as set forth in claim 1 or 2, wherein the at least one marker includes at least InDel4.

5. A method for producing a low reversion type low nornicotine content tobacco plant line, comprising:
a first step of crossing Matsukawa (Kanto), which is a variety of *Nicotiana tabacum,* and another variety of *Nicotiana tabacum* or a mutant of the another variety to obtain a first filial generation plant;
a second step of repeating backcrossing with respect to the first filial generation plant with use of *Nicotiana tabacum* other than Matsukawa (Kanto) as a backcross parent, the second step including (i) selecting, in each backcrossing, an individual having, as a heterozygote, at least one marker which is of Matsukawa (Kanto), which is selected from tables above, and which allows Matsukawa (Kanto) to be distinguished from the another variety or the mutant and the backcross parent and (ii) using the individual in next backcrossing; and
a third step of selfing an individual obtained as a result of the second step to obtain a bred progeny, and carrying out a method recited in claim 1 to select an individual having the at least one marker as a homozygote.

6. A low reversion type low nornicotine content tobacco plant obtained by a method recited in claim 5.

7. A low reversion type low nornicotine content tobacco plant (other than Matsukawa (Kanto), which is a variety of *Nicotiana tabacum*), comprising:
an endogenous CYP82E4 gene which encodes an amino acid sequence represented by SEQ ID NO. 2; and
as a homozygote, at least one marker which is selected from tables below, wherein
markers indicated by SNV are each a marker based on single nucleotide variation present at a position corresponding to each shown position on a chromosome Nt09 in a reference genome sequence of K326 of *Nicotiana tabacum,*
a "Base" indicates a base in Matsukawa (Kanto),
markers indicated by InDel are each a marker based on insertion or deletion present at a position corresponding to each shown position on the chromosome Nt09 in the reference genome sequence, and
"Insertion/Deletion" indicates whether the insertion or the deletion is present or not in Matsukawa (Kanto).
**[Table 3]**
| Marker ID | Positions on Nt09 | Base | Marker ID | Positions on Nt09 | Base | Marker ID | Positions on Nt09 | Base |
|---|---|---|---|---|---|---|---|---|
| SNV2 | 101,505,318 | T | SNV30 | 101,698,257 | G | SNV60 | 101,875,101 | A |
| SNV4 | 101,523,017 | G | SNV31 | 101,718,066 | T | SNV61 | 101,878,001 | T |
| SNV5 | 101,524,311 | T | SNV32 | 101,721,614 | C | SNV67 | 101,903,020 | T |
| SNV7 | 101,526,729 | A | SNV33 | 101,725,448 | A | SNV69 | 101,908,076 | T |
| SNV8 | 101,544,000 | G | SNV34 | 101,725,867 | C | SNV70 | 101,910,271 | T |
| SNV9 | 101,545,638 | G | SNV35 | 101,727,812 | C | SNV74 | 101,918,202 | G |
| SNV10 | 101,550,197 | A | SNV36 | 101,737,537 | G | SNV75 | 101,918,533 | T |
| SNV11 | 101,562,465 | G | SNV37 | 101,737,694 | A | SNV76 | 101,925,101 | G |
| SNV12 | 101,569,477 | T | SNV39 | 101,742,957 | T | SNV77 | 101,933,909 | T |
| SNV15 | 101,579,509 | A | SNV40 | 101,744,875 | G | SNV78 | 101,952,245 | G |
| SNV16 | 101,582,099 | G | SNV41 | 101,748,913 | T | SNV79 | 101,968,528 | A |
| SNV17 | 101,533,478 | T | SNV44 | 101,754,559 | T | SNV80 | 101,976,346 | C |
| SNV18 | 101,584,177 | T | SNV45 | 101,777,902 | A | SNV82 | 101,989,567 | A |
| SNV19 | 101,586,295 | T | SNV47 | 101,786,341 | T | SNV84 | 101,992,887 | T |
| SNV20 | 101,586,769 | C | SNV49 | 101,803,382 | A | SNV85 | 101,992,988 | C |
| SNV22 | 101,614,452 | A | SNV50 | 101,322,302 | T | SNV86 | 102,013,873 | T |
| SNV23 | 101,636,959 | T | SNV51 | 101,825,828 | A | SNV87 | 102,016,365 | T |
| SNV24 | 101,637,377 | A | SNV52 | 101,826,725 | T | SNV88 | 102,018,158 | T |
| SNV25 | 101,640,243 | C | SNV53 | 101,838,779 | G | SNV92 | 102,053,413 | C |
| SNV27 | 101,651,143 | G | SNV56 | 101,863,951 | A | SNV93 | 102,061,071 | G |
| SNV28 | 101,669,996 | T | SNV59 | 101,374,929 | C | SNV94 | 102,067,002 | A |
**[Table 4]**
| Marker ID | Positions on Nt09 | Insertion/ deletion | Marker ID | Positions on Nt09 | Insertion deletion |
|---|---|---|---|---|---|
| InDel1 | 101544714^101544775 | A insertion | InDel8 | 101870399^101870400 | TTTTTTT insertion |
| InDel2 | 101561948^101561949 | AT insertion | InDel10 | 101,881,218 | Deletion |
| InDel4 | 101685327^101685328 | GAGA insertion | InDel12 | 101,897,361 | Deletion |
| InDel5 | 101 822,299 | Deletion | InDel16 | 101953185^101953186 | T insertion |
| InDel6 | 101,855,430 | Deletion | InDel19 | 102040788..102040807 | Deletion |
| InDel7 | 101867656^101867657 | AA insertion | InDel21 | 102073969^102073970 | No insertion /deletion |
